# EUROPEAN PATENT APPLICATION

(11) **EP 2 387 986 A2**
(43) Date of publication of application: **23.11.2011**
(21) Application number: 11156548.7
(22) Date of filing: 02.03.2011
(51) Int. Cl.: A61K 8/37, A61K 8/41, A61Q 5/12, A61Q 19/00

(54) **Compositions comprising quaternary ammonium compounds and organic carbonates**

(30) Priority: 30.03.2010 US 318864 P
(71) Applicant: Evonik Goldschmidt GmbH, 45127 Essen (DE)
(72) Inventor: Smith, Ronald C, Chesterfield, VA 23832 (US); Yang, Yun, Midlothian, VA 23112 (US); Mollman, Mike Kevin, Hopewell, VA 23860 (US); Stokes Jr, Sterling M, Prince George, VA 23875 (US); Shumway, Dale F, Janesville, WI 53546 (US); Gruening, Burghard, 45134, Essen (DE)

(57) **Abstract**

The present invention is directed to compositions, preferably cosmetic compositions comprising a) at least one quaternary ammonium compound and b) at least one organic carbonate and cosmetic and hair-treatment formulations comprising the compositions according to the invention.

## Description

The invention relates to compositions comprising quaternary ammonium compounds which have a low setting point, good solubility or dispersibility in aqueous media and a high flash point and are thus highly suitable for formulating products for cosmetic, household or industrial use containing quaternary ammonium compounds.

Form JP 2003327512 a hair care cosmetic composition is known comprising specific (A) specific ether type cationic surfactants and (B) an organic solvent selected from aromatic alcohols, carbonates and specific hydroxy compounds.

Cosmetic compositions, such as, for example, hair-treatment compositions, often comprise sparingly watersoluble quaternary ammonium compounds which have a long-chain alkyl or alkenyl group.

Such compositions are usually formulated as aqueous dispersions, emulsions, microemulsions, gels or else in aerosol form and are used, for example, as shampoos, hair cures, hair rinses, etc.

For the manufacturer of such compositions, it is highly advantageous to prepare the quaternary ammonium compounds as compounded materials or formulations in the form of flakes, pellets or pastes which, as well as having a high cationic active ingredient content, have a low setting point and good solubility or dispersibility in aqueous media.

In accordance with the prior art, the above requirements can be achieved by adding short-chain alcohols, in particular isopropanol, in amounts of from 15 to 20% by weight. Because of their low boiling and flash points, however, such short-chain alcohols are problematic.

According to WO 00/28950 (US 6,638,497), the short-chain alcohols can be replaced by linear fatty alcohols (e.g. cetyl alcohol, lauryl alcohol, behenyl alcohol or stearyl alcohol). In order to lower the setting point or melting point of the mixtures to temperatures below 100 °C, glycols, such as, for example, propylene glycol or 1,3-butanediol, are additionally added. WO 00/28950 further explains that replacement of the short-chain alcohols by glycols without the simultaneous addition of fatty alcohols leads to formulations which can not be pelleted.

US 6,607,715 is directed to flakeable compositions comprising a fatty ammonium monoalkyl quat and a solvent, the solvent comprising at least one fatty alcohol and at least one glycol. To be flakeable the cationic activity can be as high as 60 %.

US 2003/0021759 is directed to a composition comprising a quaternary ammonium compound, at least one branched alcohol having 8 to 36 carbon atoms, and at least one polyhydric alcohol having from 2 to 8 carbon atoms. Preferred polyhydric alcohol is propylene glycol. This compound is explosive when mixed with air.

US 2003/0022936 is directed to a composition comprising a quaternary ammonium compound and at least one branched alcohol having 8 to 36 carbon atoms. The preferred content of the quaternary ammonium compound is of from 45 to 65 % by weight.

US 6,881,399 is directed to a compositions in form of pellets or flakes comprising 60 to 90 % by weight of a quaternary ammonium compound and at least one polyhydric alcohol having from 5 to 12 carbon atoms. In the examples dipropylene glycol or 1,6-hexanediol is used as preferred polyhydric alcohol. 75 % by weight of quaternary ammonium compounds is the maximum content that is achieved in the examples.

It was therefore an object of the present invention to provide compositions comprising a high amount of quaternary ammonium compounds and being flakeable or pelletizable. Preferable compositions preferably have a low content of volatile organic compound (VOC).

### SUMMARY OF THE INVENTION

Surprisingly, it has now been found that compositions comprising quaternary ammonium compounds and at least one organic carbonate have low setting and melting points, good solubility and dispersability in aqueous media, a high flashpoint and are flakeable or pelletizable and have a low VOC content.

Surprisingly, the compositions can be free from polyhydric alcohols, fatty alcohols and linear or branched alcohols having 8 to 36 carbon atoms and are nevertheless sufficiently hard and brittle at room temperature to allow pelletizing or flaking. The novel compositions are thus highly suitable for formulating quaternary ammonium compounds.

The invention provides compositions comprising
a) at least one quaternary ammonium compound according to formula (I) 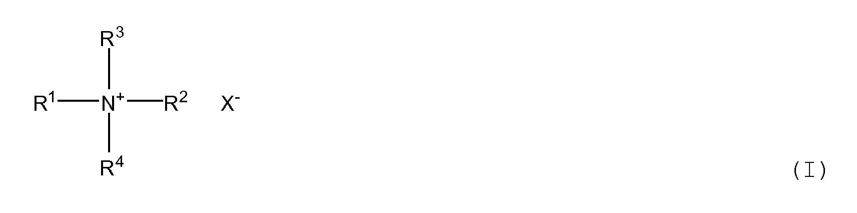 R¹ is an unbranched or branched alkyl or alkenyl group having 12 to 36 carbon atoms, a group R⁵CONH(CH₂)ₙ- or a group R⁵COO (CH₂) ₙ-, where R⁵ is an alkyl or alkenyl group having 12 to 36 carbon atoms and n is a number from 1 to 8, and
   R² and R³ independently of one another R⁴ or an unbranched or branched alkyl or alkenyl group having 12 to 36 carbon atoms, a group R⁵CONH (CH₂) ₙ- or a group
   R⁵COO (CH₂) ₙ-, where R⁵ is an alkyl or alkenyl group having 12 to 36 carbon atoms and n is a number from 1 to 8, and
   R⁴, independently of one another, may be identical or different and are a -CH₃, CH₃CH₂-, CH₃CH₂CH₂-, CH₃CH₂CH₂CH₂-, -CH₂CH₂OH, benzyl or -CH₂CH (OH) CH₂OH group and X⁻ is an anion, and
b) at least one organic carbonate.

The compositions according to the invention, their use and a process for producing the same are described below by way of example without any intention of limiting the invention to these exemplary embodiments. Where ranges, general formulae or compound classes are given below, these are intended to encompass not only the corresponding ranges or groups of compounds explicitly mentioned, but also all part ranges and part groups of compounds which can be obtained by removing individual values (ranges) or compounds. Where documents are cited within the context of the present description, then it is intended for their content, in its entirety, to form part of the disclosure of the present invention. Unless stated otherwise, all of the data in per cent (%) are per cent by weight. Unless stated otherwise, all of the average values which may be stated are number averages.

### DETAILED DESCRIPTION OF THE INVENTION

The composition according to the invention comprises:
a) at least one quaternary ammonium compound according to formula (I) 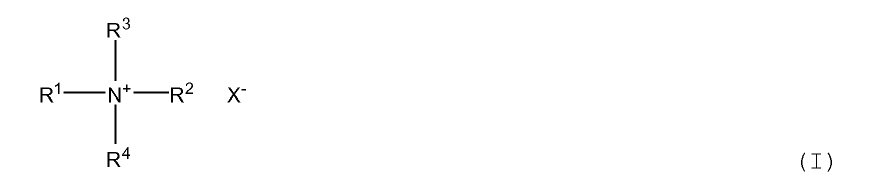 R¹ is an unbranched or branched alkyl or alkenyl group having 12 to 36 carbon atoms, a group R⁵CONH (CH₂) ₙ- or a group R⁵COO (CH₂) ₙ-, where R⁵ is an alkyl or alkenyl group having 12 to 36 carbon atoms and n is a number from 1 to 8,
   and
   R² and R³ independently of one another R⁴ or an unbranched or branched alkyl or alkenyl group having 12 to 36 carbon atoms, a group R⁵CONH (CH₂) ₙ- or a group R⁵COO (CH₂) ₙ-, where R⁵ is an alkyl or alkenyl group having 12 to 36 carbon atoms and n is a number from 1 to 8, preferably R² and R³ equal R⁴,
   and
   R⁴, independently of one another, may be identical or different and are a -CH₃, CH₃CH₂-, CH₃CH₂CH₂-, CH₃CH₂CH₂CH₂-, -CH₂CH₂OH, benzyl or -CH₂CH (OH) CH₂OH group and
   X⁻ is an anion,
   and
b) at least one organic carbonate.

The composition according to the invention is preferably free from fatty alcohols and unbranched or branched monoalcohols having 8 to 36 carbon.

The proportion (content) of quaternary ammonium compounds a), based on the finished compositions, is preferably >30 to 95% by weight, particularly preferably >40 to 90% by weight, especially preferably of from 50 to 87.5% by weight, very particularly preferably 60 to 85% by weight. Surprisingly, it has been found that the compositions can advantageously have high proportions by weight of quaternary ammonium compounds a) coupled with simultaneously low melting and setting points without the use of VOCs.

Quaternary ammonium compounds a) are preferably (C₁₂-C₃₆) -alkyltrimethylammonium compounds, particularly preferably (C₁₄-C₃₀) -alkyltrimethylammonium compounds, especially preferably (C₁₆-C₂₄) -alkyltrimethylammonium compounds. Particular preference is given to alkyltrimethylammonium compounds in which the alkyl radical is a behenyl, erucyl, cetyl or stearyl radical.

The anion X⁻ in formula (I) may be any desired charge-balancing anion; preferably chloride, iodide, bromide, methosulfate, ethosulfate, hydrogensulfate, phosphate, lactate and/or citrate, particularly preferably chloride, methosulfate and ethosulfate.

Very particularly suitable quaternary ammonium compounds a) are behenyltrimethylammonium chloride and/or behenyltrimethylammonium methosulfate.

The proportion (content) of organic carbonates b) is, based on the finished compositions, preferably of from 5 to <70% by weight, particularly preferably of from 10 to <60% by weight, especially preferably of from 12.5 to 50% by weight, very particularly preferably about 15% to 40% by weight.

Organic carbonates b) are preferably compounds of formula (II) or (III) 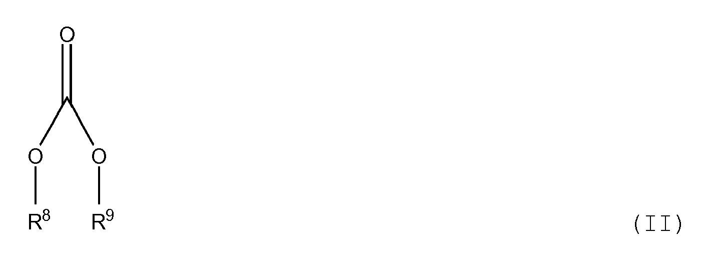 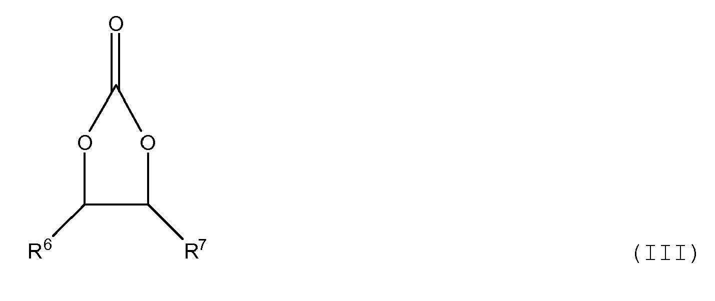 with R⁶, R⁷, R⁸, and R⁹, independently of one another, may be identical or different and are a hydrogen radical or an unbranched or branched hydrocarbyl group having 1 to 30 carbon atoms, preferably alkyl or alkenyl group having 1 to 30 carbon atoms, preferably - H, -CH₃, -CH₂OH, -CH₂CH₃ or C₆₋₂₂ aliphatic hydrocarbyl having 0 to 3 double bonds. Especially preferred organic carbonates b) are selected from the group consisting of glycerine carbonate, ethylene carbonate, propylene carbonate, polypropylene carbonate, dimethyl carbonate, diethyl hexyl carbonate, dioctyl carbonate, diethyl carbonate and fatty alcohol carbonates corresponding to formula (II) wherein R⁸ and R⁹ are C₆₋₂₂ aliphatic hydrocarbyl radicals having 0 to 3 double bonds. The fatty alcohol carbonates may be prepared by trans-esterifying diethyl carbonate.

The composition of the invention might further comprise polyhydric alcohols and/or monoalcohols having 1 to 4 carbon atoms and/or fatty alcohols.

Polyhydric alcohols are to be understood as meaning those which carry at least two OH groups in the molecule. The polyhydric alcohols according to the invention may be unbranched or branched and saturated or unsaturated. In addition, the polyhydric alcohols can be constructed from low molecular weight polyhydric alcohols linked via ether bridges.

Suitable polyhydric alcohols are preferably pentanediol, hexanediol, hexylene glycol, trimethylpentanediol, heptanediol, octanediol, nonanediol, decanediol, undecanediol, dodecanediol, diglycerol, triglycerol, dipropylene glycol, tripropylene glycol, sorbitol, xylitol, mannitol and/or mixtures thereof.

Particularly preferred polyhydric alcohols are 1,5-pentanediol, 1,2pentanediol, 1,6-hexanediol, 1,2-hexanediol, 1,7-heptanediol, 1,2-heptanediol, 1,8-octanediol, 1,2-octanediol, 1,9-nonanediol, 1,2-nonanediol, 1,10-decanediol, 1,2-decanediol, 1,11-undecanediol, 1,2-undecanediol, 1,12-dodecanediol, 1,2-dodecanediol, 2-methyl-2,4-pentanediol, 2,2,4-trimethyl-1,3-pentanediol, diglycerol, triglycerol, dipropylene glycol, tripropylene glycol, sorbitol, xylitol, mannitol and/or mixtures thereof.

Particularly advantageous application properties are exhibited by compositions which comprise at least one polyhydric alcohol having 6 to 8 carbon atoms, which can optionally be used together with other polyhydric alcohols.

Especially preferred polyhydric alcohols are ethylene glycol, propylene glycol, polypropylene glycol, butylene glycol, polyethylene glycol, 1,6-hexanediol, dipropylene glycol, 2-methyl-2,4-pentanediol, 2,2,4-trimethyl-1,3-pentanediol and mixtures thereof.

Optionally, to improve the performance properties, the compositions according to the invention may comprise unbranched or branched monoalcohols preferably having 1 to 4 carbon atoms. Preferred monoalcohols are ethanol, propanol, isopropanol, butanol, isobutanol and tert-butanol, particularly preferably isopropanol.

The composition of the invention optionally comprises fatty alcohols, preferably having from 6 to 22 carbon atoms and from 0 to 3 double bonds.

Based on the finished compositions, the compositions preferably comprise less than 5% by weight, particularly preferably less than 3% by weight, especially preferably less than 1 % by weight of such monoalcohols. In a preferred embodiment, the compositions are free from unbranched or branched monoalcohols, especially those having from 1 to 4 carbon atoms. The VOC content of such compositions is very low.

Based on the finished compositions, the compositions preferably comprise less than 5% by weight, particularly preferably less than 3% by weight, especially preferably less than 1 % by weight of polyhydric alcohols. In a preferred embodiment, the compositions are free from polyhydric alcohols.

Based on the finished compositions, the compositions preferably comprise less than 5% by weight, particularly preferably less than 3% by weight, especially preferably less than 1 % by weight of fatty alcohols. In a preferred embodiment, the compositions are free from fatty alcohols.

The compositions according to the invention preferably have setting points below 110°C, particularly preferably below 100°C, especially preferably below 95°C, very particularly preferably below 85°C.

The flash points of the compositions according to the invention are preferably above 80°C, particularly preferably above 100°C.

The compositions according to the invention may, for example, be pellets, flakes, extrudates, pastes, compacts, powders, and also emulsions or dispersions. In a preferred embodiment, the compositions according to the invention are pellets or flakes, particularly preferably pellets.

In a preferred embodiment, the compositions according to the invention are prepared by preparing a mixture comprising
i) at least one quaternary ammonium compound a), optionally containing a branched or unbranched monoalcohol having 1 to 4 carbon atoms,
ii) at least one organic carbonate b) and
iii) optionally one or more unbranched or branched monoalcohols having 1 to 4 carbon atoms and/or one or more polyhydric alcohols and/or one or more fatty alcohols.

In a preferred embodiment, the components i) to iii) are mixed and then heated, optionally with stirring. Here, the temperature is chosen so that the mixture is in the form of a melt. Preference is given to temperatures of from 70 to 120°C., particularly preferably 80 to 110 °C. In another preferred embodiment, the component i) is introduced as a melt.

The quaternary ammonium compounds a) of component i) can be prepared in a known manner by alkylation of a tertiary amine. This might be done in the presence of at least one unbranched or branched monoalcohol having 1 to 4 carbon atoms, preferably ethanol, propanol, isopropanol, butanol, isobutanol and tert-butanol, particularly preferably isopropanol. The quaternary ammonium compounds are preferably used as flakes or particularly preferably as pellets. In a preferred embodiment, the quaternary ammonium compounds comprise less than 5% by weight, preferably less than 3% by weight, more preferably less than 1 % by weight of monoalcohols.

To establish the desired content of monoalcohols in the compositions according to the invention, the components i) and/or iii) are correspondingly chosen and calculated and/or the monoalcohols are partially or completely removed from the component i) beforehand.

In a further embodiment, the monoalcohols are subsequently removed from the compositions according to the invention except for the desired residual content. The monoalcohols are preferably stripped off at 700 to 10 mbar, preferably 400 to 70 mbar, and preferably at a temperature of from 60 to 90°C.

The monoalcohols can also be distilled off at atmospheric pressure in suitable evaporation devices (e.g. thin-film evaporator) preferably at temperatures up to 120°C.

Surprisingly, it has been found that the compositions according to the invention can also be prepared "in situ" by alkylation of
i) at least one tertiary amine NR₁R₂R₃, where R₁ is an unbranched or branched alkyl or alkenyl group having 12 to 36 carbon atoms, a group R₅CONH (CH₂) ₙ- or a group R₅COO (CH₂) ₙ-, where R5 is an alkyl or alkenyl group having 12 to 36 carbon atoms and n is a number of from 1 to 8, and R₂ and R₃, independently of one another, may be identical or different and are -CH₃, -CH₂CH₃, - CH₂CH₂CH₃, -CH₂CH₂CH₂CH₃, or -CH₂CH₂ (OH) , by
ii) at least one alkylating agent chosen from
   A) R₄X, where R₄ is -CH₃, -CH₂CH₃, -CH₂CH₂CH₃, - CH₂CH₂CH₂CH₃, or -CH₂CH (OH) CH₂ (OH), and X is Cl, I, Br, OS03H or methosulfate, ethosulfate, and/or
   B) ethylene oxide and an acid HY, where Y is Cl, I, Br, OSO₃H, phosphate, citrate or lactate, in the presence of
iii) at least one organic carbonate b) and
iv) optionally at least one unbranched or branched monoalcohol having 1 to 4 carbon atoms and/or at least one polyhydric alcohol.

Preferably, in the reaction, the feed amounts of organic carbonates b) and optionally unbranched or branched monoalcohols having 1 to 4 carbon atoms and/or polyhydric alcohols are chosen to correspond to the compositions already described above as preferred for the compositions according to the invention. The contents can also be adjusted by subsequent addition or removal of the components.

In a preferred embodiment, no unbranched or branched monoalcohols having 1 to 4 carbon atoms and/or nor polyhydric alcohols are used in the reaction.

The compositions according to the invention preferably prepared by the above-described processes can be converted as homogeneous or inhomogeneous melts by cooling into pellets, flakes, extrudates or pastes or, after cooling, be further processed to give compacts, powders, granulates, emulsions or dispersions.

The compositions are preferably further processed into pellets or flakes, particularly preferably pellets.

The compositions according to the invention are generally suitable for the preparation of compositions comprising quaternary ammonium compounds.

The compositions are particularly suitable for the preparation of cosmetic, dermatological and pharmaceutical compositions.

One aspect of the invention therefore is a method for the preparation of a cosmetic formulation comprising using the composition according to the invention to prepare the cosmetic formulation. Another aspect of the invention is a cosmetic formulation comprising the composition of the invention. The cosmetic formulation is preferably a dermatological formulation or a hair-treatment formulation.

In particular, the compositions are suitable for the preparation of hair-treatment compositions.

Preferred hair-treatment formulation comprising the composition according to the invention are in the form of shampoos, rinse-off hair conditioners, cream rinses, clear rinses, hair cures, hair colorants and hair tints, permanent waving compositions, hair relaxers, leave-in hair treatments, hair gels and hair conditioners in aerosol form, spray form, foam form, fluid form, and mixtures thereof.

The invention accordingly also provides for the use of the compositions according to the invention for the preparation of compositions, preferably cosmetic, dermatological and pharmaceutical compositions, in particular hair-treatment compositions, comprising quaternary ammonium compounds.

Examples of preferred compositions are shampoos, rinse-off hair conditioners, cream rinses, clear rinses, hair cures, hair colorants and hair tints, permanent waving compositions, hair relaxers, leave-in hair treatments, hair gels, hair conditioners in aerosol, spray, foam, and fluid form, 2-in-1 shower preparations, cream shower preparations, skincare compositions, day creams, night creams, care creams, nutrient creams, sun care lotions/creams, body lotions and ointments.

The cosmetic, dermatological and pharmaceutical compositions comprise the compositions according to the invention, based on the finished compositions, preferably in amounts of from 0.1 to 15% by weight, particularly preferably 1 to 10% by weight, particularly preferably 1 to 7% by weight.

The cosmetic, dermatological and pharmaceutical compositions can comprise, as further auxiliaries and additives, all customary surfactants, oily substances, emulsifiers and coemulsifiers, cationic polymers, film formers, super fatting agents, moisture-donating agents, stabilizers, biogenic active ingredients, preservatives, pearlizing agents, dyes and fragrances, solvents, glycerol, hydrotropic agents, opacifiers, thickeners, dispersants, protein derivatives, such as gelatins, collagen hydrolyzates, natural and synthetic-based polypeptides, egg yolk, lecithin, lanolin and lanolin derivatives, silicones, deodorizing agents, substances with a keratolytic and keratoplastic action, enzymes, carrier substances, antioxidants, UV light protection filters, pigments and metal oxides, and antimicrobially effective agents.

The surfactants used may be anionic, cationic, nonionic, amphoteric and/or zwitterionic surfactants.

Preferred nonionic surfactants contain, as hydrophilic group, a polyol group, a polyolalkenyl ether group, or a combination of polyol and polyglycol ether groups. Preference is given to addition products of from 2 to 30 mol of ethylene oxide, 2 to 30 mol of ethylene oxide together with up to 5 mol of propylene oxide or of up to 5 mol of propylene oxide onto linear fatty alcohols having 8 to 22 carbon atoms and alkylphenols having 8 to 15 carbon atoms in the alkyl group, (C₁₂-C₁₉) -fatty acid mono- and diesters of addition products of from 1 to 30 mol of ethylene oxide onto glycerol, glycerol mono- and diesters and sorbitan mono- and diesters of saturated and unsaturated (C₈-C₁₈) -fatty acids and ethylene oxide addition products thereof, (C₈-C₁₈) - alkylmono- and oligoglycosides and ethoxylated analogs thereof, addition products of from 10 to 60 mol of ethylene oxide onto castor oil and hydrogenated castor oil, ethoxylated and nonethoxylated mono-, di- and trialkyl monophosphoric esters, in particular mono-, di- and tri(lauryl tetraglycol ether) o-phosphoric esters and mono-, di- and tri(cetyl tetraglycol ether) o-phosphoric esters.

Preferred amphoteric surfactants carry a (C₈-C₁₈) -alkyl or acyl group and at least one free amino group and at least one -COOH or -S03H group. Preference is given to N-acylglycines, N-alkylpropionic acid, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids having in each case 8 to 18 carbon atoms in the alkyl group. Particular preference is given to N-cocoalkylaminopropionate, cocoacylaminoethylaminopropionate and (C₁₂-C₁₈) - alkylsarcosines.

Particularly suitable zwitterionic surfactants are betaines, such as, for example, N-alkyl-N,N-dimethylammonium glycinates, e.g. cocoalkyldimethylammonium glycinates, N-acylaminopropyl-N-N-dimethylammonium glycinates, e.g. cocoacylaminopropyldimethylammonium glycinate, 2-alkyl-3-carboxymethyl-3-hydroxyethylimidazolines having in each case 8 to 18 carbon atoms in the alkyl or acyl group and cocoacylaminoethyl hydroxyethylcarboxymethyl glycinate.

The compositions preferably comprise surfactant mixtures, particular preference being given to mixtures of nonionic and zwitterionic or amphoteric surfactants in a weight ratio of from 5:1 to 1:5 or mixtures of nonionogenic surfactants and any desired mixtures of zwitterionic and amphoteric surfactants in a weight ratio of from 5:1 to 1:5.

Suitable oily substances are all known oils, fats and waxes of mineral, animal, vegetable and synthetic origin. Preference is given, as oil and fatty components, to diallyl ethers having a total of 12 to 24 carbon atoms, fatty acid esters having a total of 12 to 26 carbon atoms, liquid hydrocarbons having 10 to 32 carbon atoms and mixtures thereof.

Suitable fatty acid esters are, for example, methyl palmitate, ethyl oleate, isopropyl myristate, n-hexyl laurate, n-butyl stearate and cetyl/stearyl isononanoate.

Particular preference is given to paraffin oils, vaseline, vegetable oils, synthetic triglycerides, such as, for example, glyceryl tricaprylate, and also silicone oils.

Super fatting agents which may be used are substances such as, for example, lanolin and lecithin, and polyethoxylated or acylated lanolin and lecithin derivatives, polyol fatty acid esters, monoglycerides and fatty acid alkanolamides.

Suitable bodying agents are fatty alcohols having 12 to 22, preferably 12 to 18, carbon atoms, and also partial glycerides.

Further thickeners which may be used are polysaccharides, in particular xantham gum, guar-guar, agar-agar, alginates, carboxymethylcellulose, hydroxyethylcellulose, hydroxypropyl methylcellulose, relatively high molecular weight polyethylene glycol mono- and diesters of fatty acids, polyacrylates, polyvinyl alcohol and polyvinylpyrrolidone, surfactants such as, for example, ethoxylated fatty acid glycerides, esters of fatty acids with polyols such as, for example, pentaerythritol or trimethylpropane, fatty alcohol ethoxylates or alkyl oligoglucosides, and electrolytes, such as sodium chloride and ammonium chloride.

Examples of suitable silicone compounds are dimethylpolysiloxane, methylphenylpolysiloxanes, cyclic silicones, and amino-, fatty acid-, alcohol-, polyether-, epoxy-, fluorine- and/or alkyl-modified silicone compounds which, at room temperature, may either be in liquid form or in the form of a resin.

Biogenic active ingredients are to be understood as meaning, for example, Bisabolol(R), Allantoin(R), Phytantriol(R), Panthenol(R), AHAs, plant extracts and vitamin complexes.

Antidandruff agents which can be used are Climbazole(R), Octopirox(R), Oxiconazole(R) and Zinc Pyrethione (R) .

Customary film formers are chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof and similar compounds.

To improve the flow behavior, hydrotropic agents, such as, for example, ethanol, isopropyl alcohol, propylene glycol or glucose, can also be used.

Suitable preservatives are, for example, phenoxyethanol, formaldehyde solution, parabens, pentanediol and sorbic acid.

An available moisture-donating substance is, for example, isopropyl palmitate, glycerol and/or sorbitol.

The total content of auxiliaries and additives in the compositions is preferably 1 to 50% by weight, particularly preferably 5 to 40% by weight.

The invention will be illustrated in detail below with reference to examples and comparative examples.

### Examples:

The quat active content of the compositions according to the invention was determined by cation titration as disclosed in US 6,638,497. The setting points were determined on an Optimelt automated melting point system with the temperature increasing at a rate of 1.5°C/min.

### Example 1:

339.28 g of Adogen T2802D (behenyldimethylamine) was charged to a 1000 mL round-bottomed flask and melted with a heating mantle at 85°C. After a homogeneous melt formed, 116.6 g of dimethylsulfate was added dropwise over four hours to the melted behenyldimethylamine in the round-bottomed flask. The reaction mixture was allowed to exotherm to 105°C during the addition of dimethylsulfate. After 20% by weight of the dimethylsufate was added to the contents in the round-bottomed flask, 193.45 g of propylene carbonate was added to the solution.

At 100°C, the contents in the reaction flask was present in the completely molten form and solidified between 92-96°C. At 25°C, the composition was a wax-like solid with a quat active content of 78%.

### Example 2:

364.33 g of Adogen T2802D (behenyldimethylamine) was charged to a 1000 mL round-bottomed flask and melted with a heating mantle at 85°C. After a homogeneous melt formed, 156.3 g of diethylsulfate was added dropwise over four hours to the melted behenyldimethylamine in the round-bottomed flask. The reaction mixture was allowed to exotherm to 105°C during the addition of diethylsulfate. After 20% by weight of the diethylsufate was added to the contents in the round-bottomed flask, 218.53 g of propylene carbonate was added to the solution. At 75°C, the contents in the reaction flask was present in the completely molten form and solidified between 65-67°C. At 25°C, the composition was a wax-like solid with a quat active content of 77%.

### Example 3:

285.85 g of Adogen T2802D (behenyldimethylamine) was charged to a 1000 mL round-bottomed flask and melted with a heating mantle at 85°C. After a homogeneous melt formed, 99.42 g of dimethylsulfate was added dropwise over four hours to the melted behenyldimethylamine in the round-bottomed flask. The reaction mixture was allowed to exotherm to 105°C during the addition of dimethylsulfate. After 20% by weight of the dimethylsufate was added to the contents in the round-bottomed flask, 205.80 g of propylene carbonate was added to the solution. At 100°C, the contents in the reaction flask was present in the completely molten form and solidified between 92-95°C. At 25°C, the composition was a wax-like solid with a quat active content of 70%.

### Example 4:

338.97 g of Adogen T2802D (behenyldimethylamine) was charged to a 1000 mL round-bottomed flask and melted with a heating mantle at 85°C. After a homogeneous melt formed, 117.9 g of dimethylsulfate was added dropwise over several hours four hours to the melted behenyldimethylamine in the round-bottomed flask. The reaction mixture was allowed to exotherm to 105°C during the addition of dimethylsulfate. After 20% by weight of the dimethylsufate was added to the contents in the round-bottomed flask, 95.85 g of propylene carbonate and 95.90 g of dipropylene glycol was added to the solution. At 100°C, the contents in the reaction flask was present in the completely molten form and solidified between 90-94°C. At 25°C, the composition was a wax-like solid with a quat active content of 76%.

### Example 5:

509.5 g of Adogen T2802D (behenyldimethylamine) and 388.9 g of propylene carbonate was charged to a 2-L Parr reactor. The Parr reactor was heated to 85°C and 73.7 g of methyl chloride was added to the reactor. The temperature was allowed to rise to 115°C during the quaternization. At 100°C, the behenyltrimethylammonium chloride was present in the completely molten form and solidified between 95 to 98°C. At 25°C, the composition was a wax-like solid with a quat active content of 60%.

### Example 6:

616.0 g of Adogen T2802D (behenyldimethylamine) and 235.1 g of propylene carbonate and 235.1 g of dipropylene glycol was charged to a 2-L Parr reactor. The Parr reactor was heated to 85° and 89.1 g of methyl chloride was added to the reactor. The temperature was allowed to rise to 115°C during the quaternization. At 85°C, the behenyltrimethylammonium chloride was present in the completely molten form and solidified between 75-78°C. At 25°C, the composition was a wax-like solid with a quat active content of 60%.

### Example 7 to 11: Formulation examples of cosmetic formulations

The composition according to the invention from example 1 was melted and pelletized by being dropped onto a cold metal plate. The pellets obtained in this way are then incorporated into the corresponding formulations. The % values given in the examples 7 to 11 have all the meaning % by weight.

### Example 7: Cream rinse

| | | |
|---|---|---|
| A | Water | 90.5% |
| B | Composition from Ex. 1 | 2.5% |
| | VARISOFT® EQ 65 | 2.0% |
| | (Distearyoylethyl dimonium chloride; cetearyl alcohol) | |
| | TEGO® Alkanol 1618 | 5.0% |
| | (Cetearyl alcohol) | |
| C | Preservative | q.s. |
| | Perfume | q.s. |

The components of B is added to A and the mixture is heated to 80 to 85 °C with adequate mixing till all melted/dissolved. The mixtures is homogenized and cooled down while stirring. Add preservative and perfume when the temperature of the mixture is below 40 °C.

### Example 8: O/W handcream

| | | |
|---|---|---|
| A | Water | 75.7% |
| | Glycerin | 3.0% |
| | Composition from Ex. 1 | 6.5 |
| B | TEGOSOFT® TN2 | 3.25% |
| | (C12-15 Sodium Benzoate) | |
| | TEGOSOFT® DC | 3.0% |
| | (Decyl Oleate) | |
| | TEGOSOFT® P | 3.0% |
| | (Isopropyl Palmitate) | |
| | TEGOSOFT® SH | 2.0% |
| | (Stearyl Heptanoate) | |
| | TEGO® Alkanol 16 | 4.0% |
| | (Cetyl Alcohol) | |
| | Mineral Oil (30 mPa.s) | 0.75% |
| C | Preservative | q.s. |
| | Perfume | q.s. |

Prepare phase A in a steel container by stirring with overhead stirrer and heating to 75 to 85 °C. Phase B is prepared in a glass container by heating to 50 to 60 °C. Phase B is added to phase A and the mixture is homogenized for 2 min. The mixture is cooled down to a temperature of 40 to 45 °C with stirring before adding preservative and perfume.

### Example 9: Hair conditioner with pearlescent effect

| | | |
|---|---|---|
| A | Water | 87.5% |
| | Glycerin | 2.0% |
| B | Composition from Ex. 1 | 2.0% |
| | ABIL® Soft AF 100 | 0.5% |
| | (Methoxy PEG/PPG-7/3 aminopropyl dimethicone) | |
| | TEGO® Alkanol 16 | 5.0% |
| | (Cetyl alcohol) | |
| C | TEGO® Pearl N 100 | 3.0% |
| | Preservative | q.s. |
| | Perfume | q.s. |

The components of phase A are blended and the blend is heated to 80 to 85 °C. Phase B is added to A with adequate mixing. The temperature is hold till all components are melted/dissolved. The mixture is homogenized and cooled down while stirring. Phase C is added with adequate mixing when the temperature of the mixture is below 45 °C.

### Example 10: Leave-in conditioner

| | | |
|---|---|---|
| A | TEGINACID® C | 4.0% |
| | (Cetearth-25) | |
| | ABIL® OSW 5 | 20.0% |
| | ABIL® Soft AF 100 | 1.0% |
| | (Methoxy PEG/PPG-7/3 aminopropyl dimethicone) | |
| | TEGO® Alkanol L 4 | 0.5% |
| | (Laureth-4) | |
| | Composition from Ex. 1 | 0.5% |
| B | Water | 68.5% |
| | Propylene Glycol | 5.0% |
| | TEGO® Carbomer 340 FD | 0.5% |
| C | NaOH (25% aqueous solution) | q.s. |
| | Preservative | q.s. |
| | Parfume | q.s. |

TEGO® Carbomer 340 FD is dissolved in water and propylene glycol is added. Phase A and phase B are heated separately to 80 ― 85 °C. Both phases are combined and the mixture is homogenized. Subsequently the mixture is neutralized with NaOH solution as required and homogenized again. The mixture is cooled down while stirring. Preservative and perfume are added at a temperature of the mixture below 45 °C.

### Example 11: Hot Oil Treatment

| | | |
|---|---|---|
| A. | Water | 96.0% |
| | Polyquaternium-10 | 1.0% |
| | Hydroxyethylcellulose | 0.5% |
| B | Composition from Ex. 1 | 1.5% |
| | Cocamide DEA | 1.0% |
| C | Citric acid (25% aqueous solution) | q.s. |
| | Preservative | q.s. |
| | Perfume | q.s |

Pre-weighted polyquaternium-10 and hydroxyethylcellulose are sprinkled into water while stirring. The agitation is continued until phase A is clear. Phase B is pre-mixued until a uniform gel is formed. Phase A is heated to 65 to 70 °C. Phase B is added to phase A with gentle agitation. This mixtures is cooled down to room temperature with mixing. The pH is adjusted to 4.5 to 5.5 with an aqueous citric acid solution.

## Claims

1. A composition comprising
a) at least one quaternary ammonium compound according to formula (I) 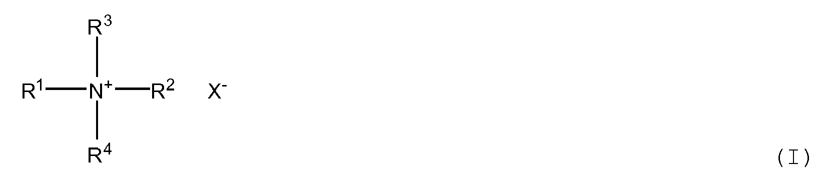 R¹ is an unbranched or branched alkyl or alkenyl group having 12 to 36 carbon atoms, a group R⁵CONH (CH₂) ₙ- or a group R⁵COO (CH₂) ₙ- , where R⁵ is an alkyl or alkenyl group having 12 to 36 carbon atoms and n is a number from 1 to 8,
and
R² and R³ independently of one another R⁴ or an unbranched or branched alkyl or alkenyl group having 12 to 36 carbon atoms, a group R⁵CONH (CH₂) ₙ- or a group R⁵COO (CH₂) ₙ-, where R⁵ is an alkyl or alkenyl group having 12 to 36 carbon atoms and n is a number from 1 to 8,
and
R⁴, independently of one another, may be identical or different and are a -CH₃, CH₃CH₂-, CH₃CH₂CH₂-, CH₃CH₂CH₂CH₂-, -CH₂CH₂OH, benzyl, or -CH₂CH (OH) CH₂OH group and
X⁻ is an anion,
and
b) at least one organic carbonate.

2. The composition as claimed in claim 1, wherein a content of quaternary ammonium compounds a), based on a finished composition, is >30 to 95% by weight.

3. The composition as claimed in claim 1 or 2, wherein the quaternary ammonium compounds a) are (C₁₂-C₃₆) - alkyltrimethylammonium compounds.

4. The composition as claimed in at least one of claims 1 to 3, wherein the anion X in formula (I) is selected from the group consisting of chloride, iodide, bromide, methosulfate, ethosulfate, hydrogensulfate, phosphate, lactate, citrate, and mixtures thereof.

5. The composition as claimed in at least one of claims 1 to 4, wherein the quaternary ammonium compound a) is behenyltrimethylammonium methosulfate or behenyltrimethylammonium chloride.

6. The composition as claimed in at least one of claims 1 to 5, wherein a content of organic carbonates b), based on the finished composition, is of from 5 to <70 % by weight.

7. The composition as claimed in at least one of claims 1 to 6, wherein the organic carbonates b) are compounds of formula (II) or (III) 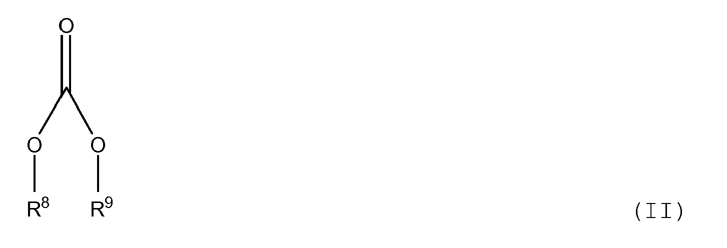 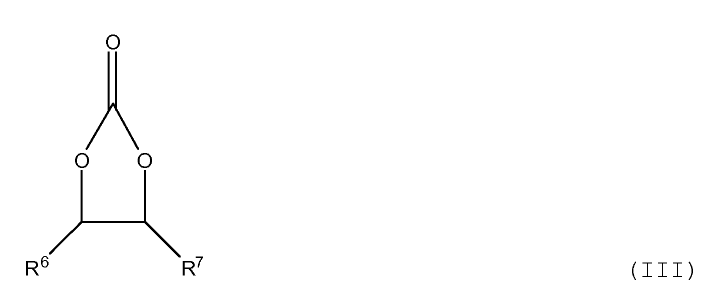 with R⁶, R⁷, R⁸, and R⁹, independently of one another, may be identical or different and are a hydrogen radical or an unbranched or branched hydrocarbyl group having 1 to 30 carbon atoms.

8. The composition as claimed in at least one of claims 1 to 7, wherein the organic carbonates b) are selected from the group consisting of glycerin carbonate, ethylene carbonate, propylene carbonate, polypropylene carbonate, dimethyl carbonate, diethyl hexyl carbonate, dioctyl carbonate, diethyl carbonate and fatty alcohol carbonates corresponding to formula (II) wherein R⁸ and R⁹ are C₆₋₂₂ aliphatic hydrocarbyl radicals having 0 to 3 double bonds.

9. The composition as claimed in at least one of claims 1 to 8, which is free from fatty alcohols and unbranched or branched monoalcohols having 8 to 36 carbon atoms.

10. The composition as claimed in at least one of claims 1 to 9, further comprising polyhydric alcohols and/or monoalcohols having 1 to 4 carbon atoms and/or fatty alcohols.

11. The composition as claimed in at least one of claims 1 to 10, which comprises, based on a finished composition, less than 5% by weight, of unbranched or branched monoalcohols having 1 to 4 carbon atoms and/or of polyhydric alcohols and/or of fatty alcohols.

12. The composition as claimed in at least one of claims 1 to 11, which has a setting point below 100 °C.

13. The composition as claimed in at least one of claims 1 to 12, which is in a form selected from the group consisting of pellets, flakes, extrudates, pastes, compacts, powders, emulsions, dispersions, and mixtures thereof.

14. A process for the preparation of the composition of at least one of claims 1 to 13, which comprises preparing a mixture comprising
i) at least one quaternary ammonium compound a), and
ii) at least one organic carbonate.

15. A method for the preparation of a cosmetic formulation comprising using the composition of one of claims 1 to 13 to prepare the cosmetic formulation.

16. A cosmetic formulation comprising the composition of at least one of claims 1 to 13.

17. The cosmetic formulation of claim 16, wherein the cosmetic formulation is a dermatological formulation or a hair-treatment formulation.

18. Hair-treatment formulation comprising the composition of claim 1 in the form of shampoos, rinse-off hair conditioners, cream rinses, clear rinses, hair cures, hair colorants and hair tints, permanent waving compositions, hair relaxer, leave-in hair treatments, hair gels and hair conditioners in aerosol form, spray form, fluid form, and mixtures thereof.
